# EUROPÄISCHE PATENTSCHRIFT

(11) **EP 2 616 446 B1**
(45) Veröffentlichungstag und Bekanntmachung des Hinweises auf die Patenterteilung: **29.04.2015**
(21) Anmeldenummer: 11761030.3
(22) Anmeldetag: 05.09.2011
(51) Int. Cl.: C07D 233/54

(54) **NEUE IMIDAZOLIUMSALZE UND DARAUF BASIERENDE CARBEN-METALLKOMPLEXE ZUR VERWENDUNG ALS BIOANALYTISCHE MARKER FÜR BIOMOLEKÜLE**
NEW IMIDAZOLIUM SALTS AND CARBENE METAL COMPLEXES THEREOF FOR USE AS BIOANALYTICAL MARKERS FOR BIOMOLECULES
NOUVEAUX SELS D'IMIDAZOLIUM ET LEURS COMPLEXES CARBÈNE-MÉTAL POUR L'UTILISATION COMME MARQUEURS BIOANALYTIQUES POUR BIOMOLÉCULES

(30) Priorität: 15.09.2010 DE 102010040822
(43) Veröffentlichungstag der Anmeldung: 24.07.2013
(73) Patentinhaber: Ernst-Moritz-Arndt-Universität Greifswald, 17487 Greifswald (DE)
(72) Erfinder: KÜHL, Olaf, 17489 Greifswald (DE)
(74) Vertreter: Wablat Lange Karthaus
(86) Internationale Anmeldenummer: PCT/EP2011/065266
(87) Internationale Veröffentlichungsnummer: WO 2012/034880

(56) Entgegenhaltungen:
- US-A- 5 874 587

## Beschreibung

Die vorliegende Erfindung betrifft Imidazoliumsalze, die entsprechenden Carben-Metallkomplexe sowie deren Verwendung als bioanalytische Marker für Biomoleküle.

Die Erforschung der Struktur und Funktionsweise von Biomolekülen wie Proteinen und Kohlenhydraten, sowie von Genomen und Gentranskripten erfordert entweder spezielle Derivate oder/und massenspektrometrische Analysen. Eingesetzt werden beispielsweise Methoden wie ESI (Elektrospray) [1], MALDI (matrix-assisted laser desorption and ionisation) [2] und ICP-MS (inductively coupled plasma-mass spectrometry) [3-5]. Solche Verfahren bedingen Separations- und Reinigungsschritte mittels Flüssigkeitschromatographie (LC) oder Hochleistungsflüssigkeitschromatographie (HPLC) [6]. Dies erfordert Techniken, die eine Kombination aus Reinigung und Analyse darstellen und üblicherweise mit einem Akronym bezeichnet werden, welches die Namen der einzelnen Techniken vereint (hyphenated techniques).

Die Struktur und der sequentielle Aufbau der genannten Biomoleküle stellen große Anforderungen an die quantitative Bestimmung. Insbesondere erschwert das Fehlen von quantitativ messbaren funktionellen Gruppen bzw. die eingeschränkte Verfügbarkeit geeigneter Heteroatome die Verwendung von Analysemethoden wie Fluoreszenzspektroskopie oder UV/vis-Spektroskopie, sowie den Einsatz elektroanalytischer Verfahren. Sogar die ICP-MS wird ohne einen zusätzlichen Derivatisierungsschritt erschwert bzw. unmöglich [6]. Häufig erfolgt ein sogenannter Derivatisierungsschritt, wodurch eine chromophore, fluorophore oder elektroaktive Gruppe in den Analyten eingebracht wird. Die Einbringung eines Heteroelements als entsprechender Derivatisierungsschritt eröffnet den Zugang zu ICP-MS-Studien [7].

Zu verschiedenen medizinisch-chemischen Anwendungen wurden für die routinemäßige Bestimmung bestimmter Analyte initiale Derivatisierungsschritte entwickelt und in die jeweiligen Protokolle aufgenommen. Der größte Nachteil hierbei ist die Beschränkung auf die gleichzeitige Detektion lediglich eines einzelnen Analyten. Für die Bestimmung mehrerer Analyte sind multiple Detektionssequenzen notwendig. Die gleichzeitige Bestimmung ist derzeit unmöglich. Protokolle für die simultane Bestimmung mehrerer Analyte, vorzugsweise bei Anwendung identischer Analysemethoden, sind daher dringend erforderlich [8].

Im Bereich der Proteomik ist die gleichzeitige Messung verschiedener Analyte anhand von Tandem Mass Tags (TMT) oder mittels isobarer Datenerkennung (isobaric tagging) zur relativen und absoluten Quantifizierung (iTRAQ) [9,10] möglich. Isobare Tagging-Techniken bedingen allerdings die Markierung mit schweren Isotopen (²D, ¹³C, ¹⁵N) in einer kostspieligen mehrschrittigen Synthese, obwohl die Marker kommerziell erhältlich sind.

Markierungsmethoden sollten stabile Marker bieten, die eine niedrige Detektionsgrenze erlauben und in den eingesetzten Mengen nicht-toxisch und kostengünstig sind. Weiterhin sollten sie quantitativ und spezifisch einzubringen sein. Eine breite Palette individueller Marker sollte zugänglich sein, wodurch die gleichzeitige Messung verschiedener Analyte möglich wäre.

Organometallische Verbindungen bzw. Metallkoordinationsverbindungen bieten das Potential, alle genannten Anforderungen zu erfüllen. Mehrere Verbindungsklassen wurden bereits synthetisiert und haben als bioanalytische Marker (bioanalytical tag, BAT) Verwendung gefunden [6]. "Stabil" bedeutet in diesem Sinne, dass die Verbindung stabil gegen Luft und Feuchtigkeit ist und für Koordinationsverbindungen und organometallische Verbindungen gleichermaßen nutzbar ist. "Stabil" meint aber auch, dass Stabilität gegenüber Auswaschung oder allgemein gegenüber Entfernung vom Biomolekül gegeben ist. Im Fall von metallhaltigen BATs bedeutet dies eine starke Koordination des Liganden / der Liganden zum Metall, was am besten mittels einer Kombination aus Chelatliganden und N-heterozyklischen Carbenen (NHC) möglich sein sollte [11,12].

Viel versprechende Verbindungsklassen, die für BAT-Anwendungen nutzbar sind, sind Metallcarbonyle, Carbenkomplexe (Fischer und Wanzlick-Arduengo), sowie Ferrocene und Chelatkomplexe [6]. Allerdings sind Metallcarbonyle in der Massenspektrometrie anfällig für einen frühen Carbonyl-Verlust, wodurch die Spektrenauswertung erschwert wird [13]. Andererseits bieten sie ein einzigartiges Detektionsfenster für die IR-Spektroskopie [14]. Die am weitesten entwickelte Verbindungsklasse sind eindeutig die Ferrocene für die elektrochemische Detektion. Allerdings bestehen auch hierbei im Hinblick auf Derivatisierung und entsprechend im Hinblick auf die multiple Bestimmung von Analyten Beschränkungen [15].

Insgesamt sind selbst Ferrocene nicht in der Lage, gleichzeitig die Vorzüge der elektrochemischen Detektion, einzigartige Isotopenmuster für die MS-Detektion und einen breiten Modifizierungsbereich auf sich zu vereinen.

Entsprechend ist es die Aufgabe der vorliegenden Erfindung, Verbindungen bereitzustellen, welche die oben genannten Nachteile überwinden.

Die Aufgabe wird gelöst mit Imidazoliumsalzen gemäß den Ansprüchen 1 und 5 sowie mit entsprechenden Carben-Metallkomplexen gemäß den Ansprüchen 6 und 10. Weitere bevorzugte Ausführungsformen ergeben sich aus den Unteransprüchen.

In anderen Worten wird die Aufgabe durch Imidazoliumsalze der allgemeinen Formel I gelöst, 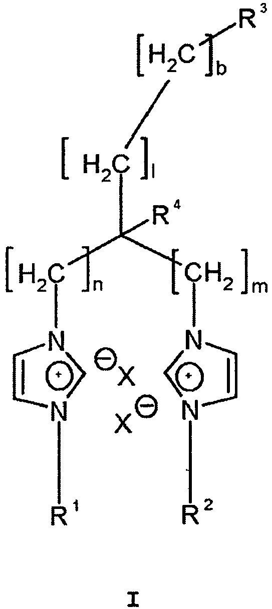 wobei b, l, m, n und gegebenenfalls q gleich oder verschieden sind und jeweils unabhängig voneinander Null oder 1 sind; und
- X⁻: ein Anion ist;
- R¹ und R²: gleich oder verschieden sind und offenkettig, verzweigt oder unverzweigt oder als Bestandteil eines Ringes oder Ringsystems vorliegen und ausgewählt sind aus der Gruppe von Wasserstoff, C₁-C₁₂-n-Alkylrest, C₃-C₁₂-verzweigter Alkylrest, C₃-C₁₂-Cycloalkylrest, C₂-C₁₂-Alkenylrest, C₇-C₂₀-Arylalkylrest, C₅-C₁₄-Arylrest,
welcher seinerseits gleich oder unterschiedlich substituiert ist durch einen oder mehrere verzweigte oder unverzweigte C₁-C₃-Alkylreste, wobei die cyclischen und aromatischen Systeme eingliedrige Ringe oder mehrgliedrige annelierte oder isolierte Ringe sind oder mit dem Imidazolring der Struktur gemäß Formel I ein Ringsystem bilden;
- R³: ausgewählt ist aus der Gruppe von -NH₂, -COOH, -CONH₂ und -COOR"-Rest, wobei R" ein C₁-C₆-n-Alkylrest ist;
- R⁴: Wasserstoff oder R⁵ist, wobei R⁵ ein Imidazolring der Formel II ist, 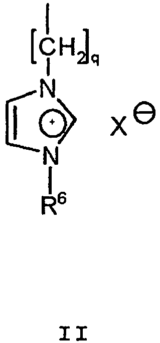 wobei q Null oder 1 ist;
- R⁶: gleich oder verschieden zu R¹ und R² und aus der gleichen Gruppe wie R¹, R² ausgewählt ist.

Das Anion X⁻ ist keinen Beschränkungen unterworfen, d.h. es kann ein beliebiges Anion sein, welches die insgesamt neutrale Ladung des Komplexes gewährleistet. Vorzugsweise zu nennen sind hier die Halogenide wie Fluorid, Chlorid und Bromid, aber auch andere einwertige Anionen wie Nitrate, BF₄⁻, BPh₄⁻, PF₆⁻, SbF₆⁻ und Sb₂F₁₁⁻, sowie zwei- oder dreiwertige Anionen wie Sulfate, Phosphate.

Vorzugsweise sind die Reste R¹, R² und gegebenenfalls R⁶ gleich oder verschieden und ausgewählt aus der Gruppe der C₁-C₆-n-Alkylreste, vorzugsweise aus der Gruppe der C₁-C₃-n-Alkylreste. Besonders bevorzugt sind R¹, R² und gegebenenfalls R⁶ Methylreste.

Von den Indizes b, l, m, n und gegebenenfalls q sind vorzugsweise n, m und gegebenenfalls q Null oder 1 sowie b und I Null.

Der Rest R³, welcher die funktionelle Gruppe für eine spätere Verlinkung zum jeweiligen Biomolekül darstellt, ist ausgewählt aus -NH₂, -COOH, -CONH₂ und -COOR"-Rest, wobei R" ein C₁-C₆-n-Alkylrest, vorzugsweise ein Methyl- oder Ethylrest, ist.

Exemplarische Vertreter für besonders bevorzugte Imidazoliumsalze sind nachfolgend in Schema 1 a abgebildet. 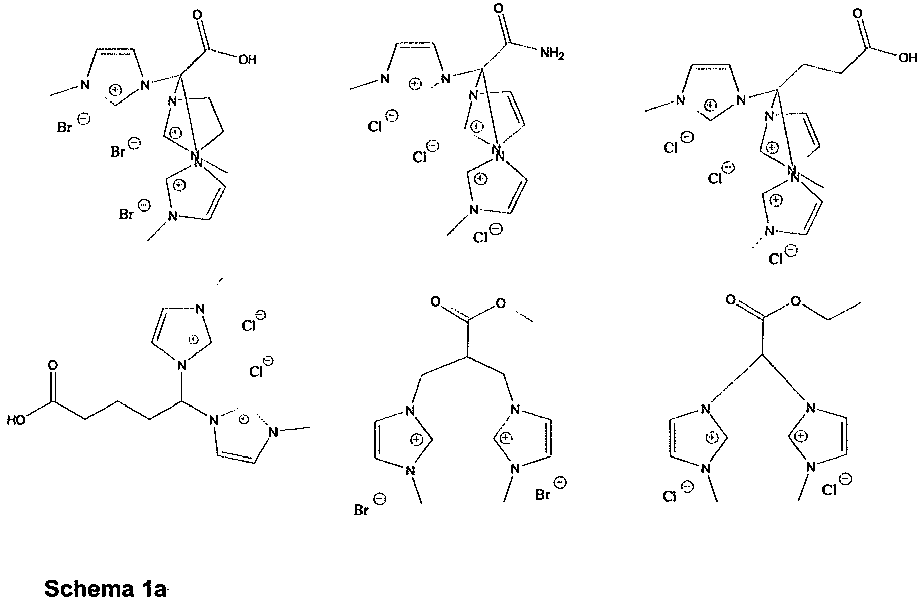 Schema **1a**

Ein weiterer bevorzugter Vertreter ist in Schema 1 b abgebildet.

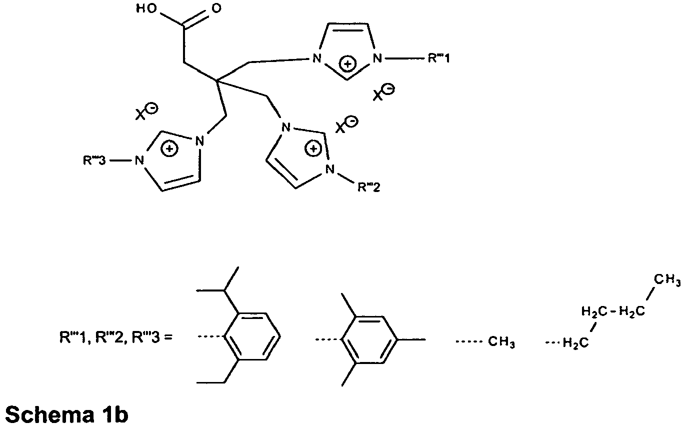

Die Reste R'"1, R"'2 und R'"3 gemäß Schema 1b sind jeweils gleich oder verschieden und unabhängig voneinander ausgewählt aus 1-(2,6-Di-isopropylphenyl), 1-(2,4,6-Trimethylphenyl), Methyl und n-Butyl wie in Schema 1b gezeigt. Das Anion X⁻ ist keinen Limitationen unterworfen. Vorzugsweise sind die Reste R'"1, R'"2 und R'"3 gleichartig, d.h. besonders bevorzugte Vertreter der Imidazolium-salze aus Schema 1 b sind in Schema 1 c gezeigt, wobei X⁻ vorzugsweise F⁻, Cl⁻oder Br⁻ ist.

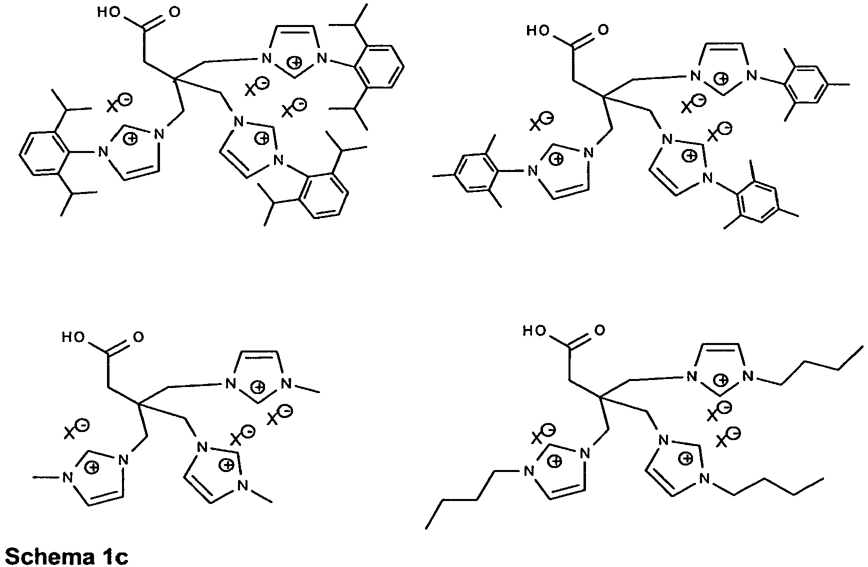

Die Kombination zweier funktionalisierter Bis-Carbene zu einer einzigen Tetrakis-Carbeneinheit ist ebenfalls Gegenstand der vorliegenden Erfindung. Grundlage hierfür sind die Imidazoliumsalze gemäß der allgemeinen Formel III, welche im Nachfolgenden als verbrückte Imidazoliumsalze bezeichnet werden.

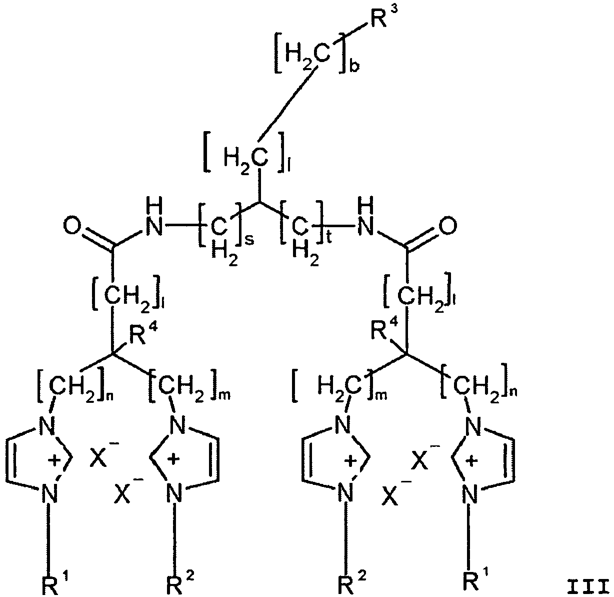

R¹ bis R⁴ sowie b, l, m, n haben bei den verbrückten Imidazoliumsalzen die gleiche Bedeutung, wie sie im Vorherigen zu den Imidazoliumsalzen genannt wurde. Weiterhin sind die Indizes s und t gleich oder verschieden und jeweils unabhängig voneinander Null oder eine ganze Zahl von 1 bis 3.

Die Darstellung solcher verbrückter Imidazoliumsalze erfolgt vorzugsweise durch Kopplung zweier bidentaler Einheiten mittels eines [2+1]-funktionalen Linkers, wie sie exemplarisch in Schema 2 dargestellt ist.

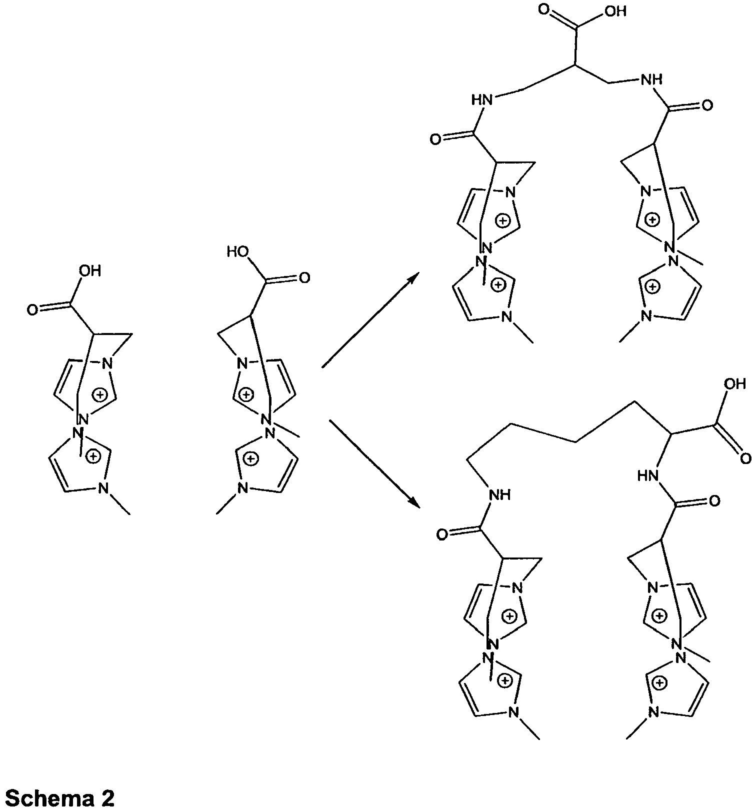

Die erfindungsgemäßen Imidazoliumsalze und verbrückten Imidazoliumsalze werden im Weiteren zu N-Heterocyclischen Carbenkomplexen umgesetzt.

Gegenstand der Erfindung sind somit auch Carben-Metallkomplexe der allgemeinen Formel IV 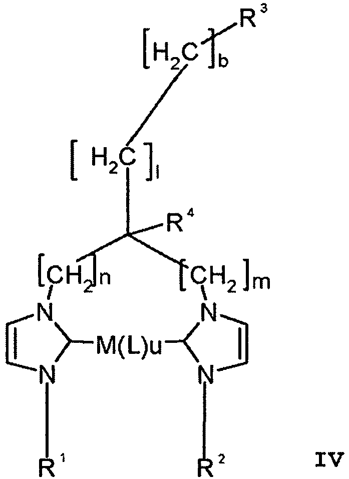 wobei R¹ bis R⁴ und gegebenenfalls R⁶ und b, l, m, n und gegebenenfalls q und r die bereits zu den Imidazoliumsalzen erläuterte Bedeutung haben und M ein Metall der 6.-12. Gruppe des Periodensystems , L ein Ligand und u eine ganze Zahl von 1 bis 4 ist, wobei bei u = 2, 3 oder 4 die Liganden L gleich oder verschieden sind und mehrere Liganden L gegebenenfalls miteinander einen zwei- oder mehrzähnigen Chelatliganden bilden und wobei bei R⁴= Imidazolring gemäß Formel II der Imidazolring ebenfalls an das Metall M koordiniert.

Vorzugsweise ist das Metall M aus der Gruppe der Metalle Kupfer, Eisen, Ruthenium, Nickel und Palladium oder aus der Gruppe der Metalle Technetium, Rhenium und Kobalt ausgewählt.

Der Ligand L ist ausgewählt aus der Gruppe von
CO;
Nitril;
Isonitril;
Nitrosyl;
Halogenidion;
Wasserstoffatom;
C₁-C₁₂-Alkylanion, Allylanion, Methallylanion, Benzylanion, C₆-C₁₄-Arylanion, C₁-C₁₂-Alkoxyanion, C₆-C₁₄-Aryloxyanion, C₁-C₁₂-Heteroalkylanion, C₆-C₁₄-Heteroaryl-anion, C₁-C₁₂-Heteroalkoxyanion, C₆-C₁₄-Heteroaryloxyanion oder C₆-C₁₄-Heteroaryloxyanion, welches unsubstituiert ist oder gleich oder unterschiedlich substituiert ist durch ein oder mehrere C₁-C₁₂-Alkylreste oder C₁-C₁₂-Heteroalkylreste, wobei die Heteroatome aus der Gruppe von B, Al, Ga, In, N, P, As, Sb, Bi, Si, Ge, Sn, Pb, O, S, Se und Te ausgewählt sind und wobei die Anionen unsubstituiert oder ganz oder teilweise mit einem oder mehreren, gleichen oder unterschiedlichen Heteroatomen aus der selben Gruppe in Form funktioneller Gruppe(n) substituiert sind, wobei die cyclischen und aromatischen Systeme eingliedrige Ringe oder mehrgliedrige annelierte oder isolierte Ringe sind;
primäres, sekundäres, tertiäres Amin;
primäres, sekundäres, tertiäres Phosphan;
primäres, sekundäres, tertiäres Phosphit oder Carben.

Der Ligand L kann ein Chelatligand sein. Vorzugsweise ist der Ligand L ausgewählt aus der Gruppe der Halogenidionen, besonders bevorzugt sind hierbei Bromid oder Chlorid.

Die Einheit aus Übergangsmetall und Carben ermöglicht den Einsatz für die quantitative Detektion auf der Basis von IR, UV-vis, Fluoreszenzspektroskopie, ICP-MS oder auf Basis elektrochemischer Methoden, gegebenenfalls mit Hilfe eines radioaktiven Indikators. Sie kann als Detektionseinheit bezeichnet werden. Die funktionelle Gruppe R³ ermöglicht die Kopplung der Detektionseinheit an das jewielige Biomolekül. Durch Veränderung des Metalls und/oder des Metall-Coliganden (Ligand L) kann die Detektionsart oder die Position des Detektionssignals variiert werden. Dies bedeutet, dass die Auswahl der Detektionsart in die Hand des Anwenders gelegt wird und nicht mehr der limitierende Faktor ist. Die Veränderung der Signalposition innerhalb der Detektionsart ermöglicht es, mehrere BATs simultan zu messen, d.h. eine Analyse mehrerer Analyten in einem Schritt ist auf diesem Weg machbar.

In Schema 3 ist beispielhaft eine Serie verschiedener aber ähnlicher Molybdän-Carbonyl-Carben-Komplexe gezeigt, deren A1-Streckschwingungsfrequenz im IR-Spektrum in Abhängigkeit von der Zähnigkeit des funktionalisierten NHC-Liganden variiert [14].

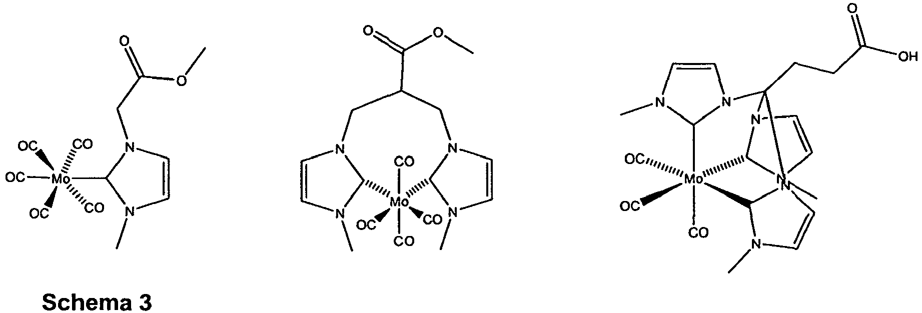

Für elektrochemische Detektionsverfahren werden oft Rutheniumkomplexe eingesetzt. Insbesondere kann das Redoxpaar Ru(II)/Ru(III) nahezu genauso generell verwendet werden wie das Fe(II)/Fe(III)-Redoxpaar der Ferrocene. Letzteres kann nur moderat verändert werden, indem die Substituenten am Cyclopentadienylring modifiziert werden. Dies ist synthetisch schwierig und limitiert im Hinblick auf den tatsächlichen Shift des Signals. Im Gegensatz dazu ist die Modifikation der erfindungsgemäßen Ruthenium-NHC-Komplexe einfach. Einen Überblick über Fe-, Ru-Komplexe bietet Schema 4.

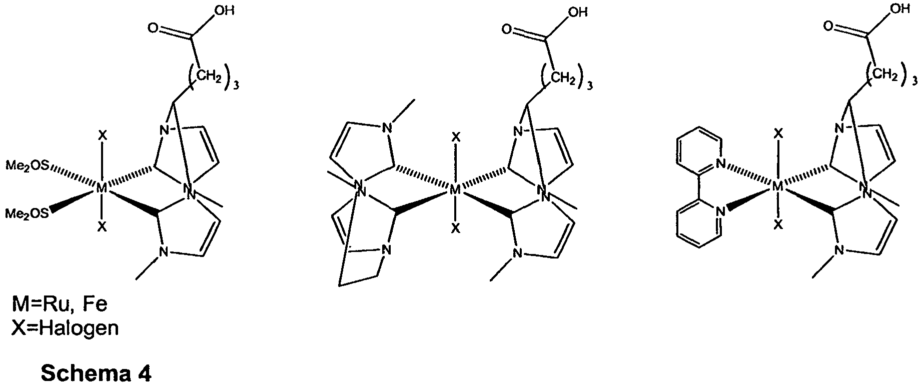

Hinsichtlich ICP-MS kann eine Variation des Metallatoms genutzt werden, um verschiedene Analyten quantitativ zu identifizieren. Durch Nutzung der verschiedenen Isotopenmuster der Metallatome kann die gleichzeitige Messung verschiedener Analyte allein mittels Massenspektrometrie erfolgen. Verschiedene hierfür nutzbare Carben-Metallkomplexe sind in Schema 5 abgebildet.

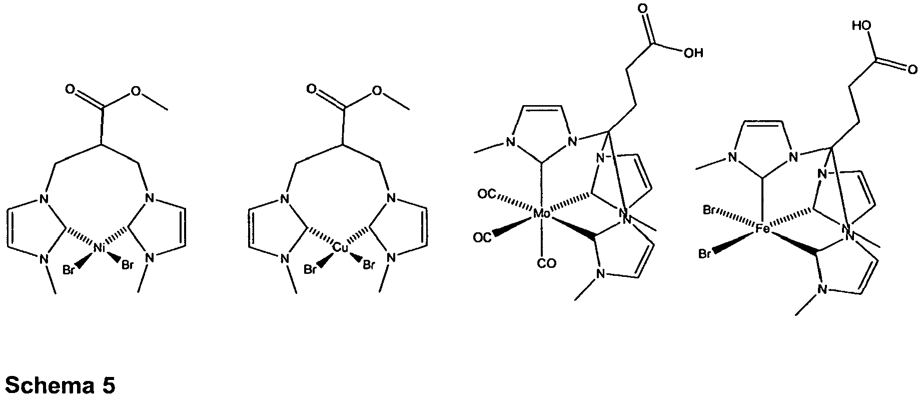

Besonders bevorzugte Carben-Metallkomplexe (BATs), welche erfindungsgemäß zum Einsatz kommen, sind nachfolgend in Schema 6 abgebildet.

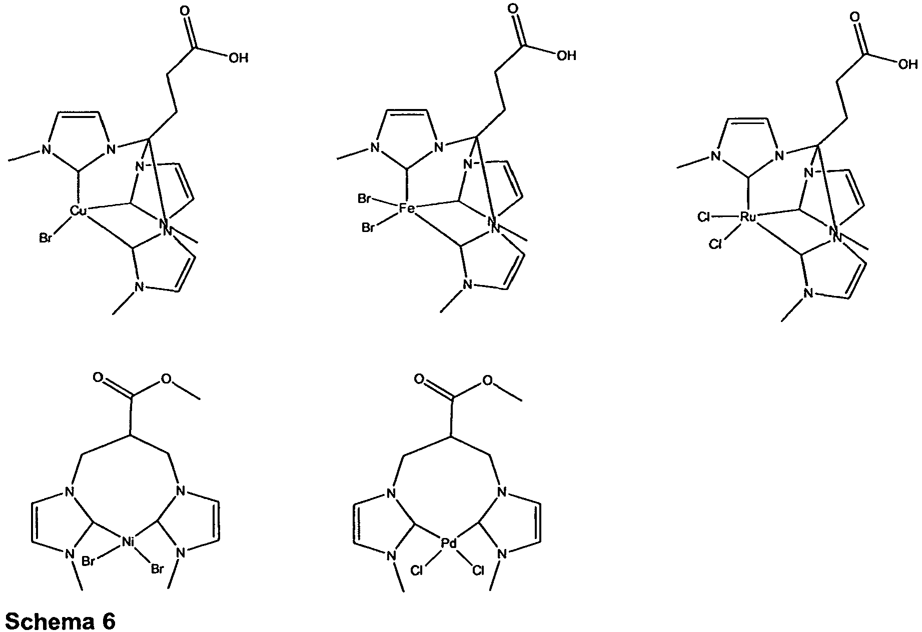

Entsprechend den oben genannten Imidazoliumsalzen der allgemeinen Formel III sind auch Carben-Metallkomplexe der allgemeinen Formel V Gegenstand der vorliegenden Erfindung. 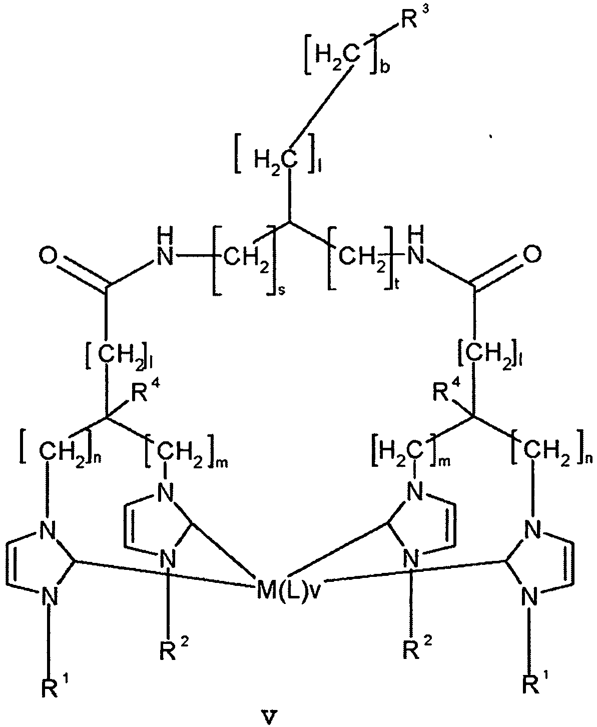 wobei R¹ bis R³ und b, l, m und n sowie M und L die vorher genannte Bedeutung haben, R⁴ Wasserstoff ist und v Null, 1 oder 2 ist. Sie werden als verbrückte Carben-Metallkomplexe bzw. kombinatorische BATs bezeichnet. Solche kombinatorischen BATs sind schwieriger in der Erzeugung aufgrund der positiven Ladung der einzelnen Imidazoliumringe. Als Lösung wird zur Synthese vorzugsweise ein sequentieller Ansatz verwandt, wobei zwei zweizähnige Einheiten mittels eines [2+1]-funktionalen Linkers zu einem vierzähnigen Liganden verbunden werden, wie es oben in Schema 2 dargestellt ist. Diese Gruppierung wird anschließend zum Carben-Metallkomplex umgesetzt. Einen Gesamtüberblick bietet Schema 7.

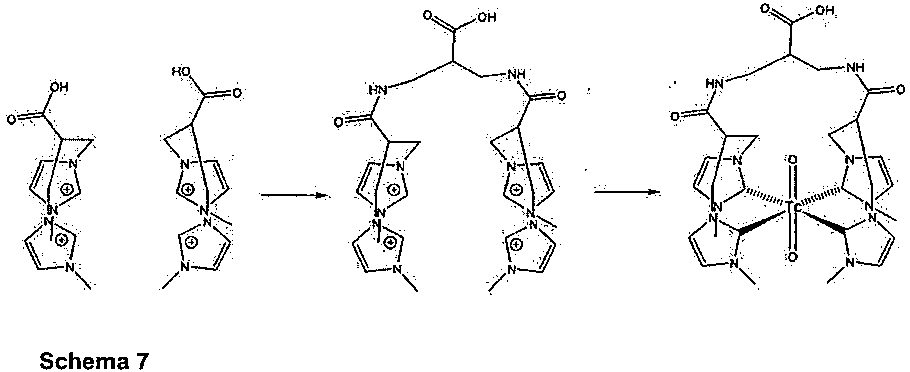

Im Sinne der Erfindung können auch zuerst zwei zweizähnige Liganden zu einem tetrakis-Carben Metallkomplex umgesetzt werden und dann erst die beiden Einheiten verlinkt werden. Ebenso kann eine funktionalisierte und eine nichtfunktionalisierte Bis-Carben Einheit zur Synthese dieses BAT verwendet werden.

Bei einem Carben-Metallkomplex mit einem vierzähnigen Liganden wie abschließend in Schema 7 dargestellt, ist das Metall hydrophob verkapselt. In dieser Form ist der Transfer durch hydrophobe Barrieren wie beispielsweise Zellmembranen möglich. Verwendet werden die Carben-Metallkomplexe, d.h. sowohl die einfachen als auch die verbrückten Carben-Metallkomplexe, als bioanalytische Marker für Biomoleküle, wobei die Metallkomplexe über die funktionelle Gruppe des Restes R³ kovalent an eine entsprechende funktionelle Gruppe des Biomoleküls, vorzugsweise eine Hydroxyl-, Thiol- oder Amino-Gruppe, besonders bevorzugt über eine Thiol- oder Amino-Gruppe, gebunden sind.

Das jeweils zu analysierende Biomolekül entstammt der Gruppe von Proteinen, Peptiden, Kohlenhydraten, Nukleinbasen (Cytosin, Uracil, Thymin, Guanin, Adenin, Hypoxanthin), DNA und RNA sowie Hybriden unter Beteiligung eines oder mehrerer Mitglieder dieser Gruppe.

Verschiedene funktionelle Gruppen des Biomoleküls können gezielt angesteuert werden, indem die Linkereinheit des BAT modifiziert wird. Als Linkereinheit wird die Gruppierung [CH₂]ₗ-Rₐ-[CH₂]_{b}- R³ verstanden. Erfindungsgemäß bevorzugt werden Aminfunktionalitäten wie beispielsweise bei Lysin oder der N-Terminus von Peptiden angesteuert. Für letzteres wird beispielsweise ein N-Succinimidester als Linker verwendet. Für die Ankopplung an Amine können beispielsweise Carbonylgruppen als Rest R³ verwendet werden. Weitere ansteuerbare funktionelle Gruppen sind Thiolgruppen z.B. in den Cystein-Seitenketten von Peptiden, wobei beispielsweise N-Succinimid oder lodacetamid als Linker Verwendung finden. Für Molybdänkomplexe sind einige der erfindungsgemäß bevorzugten Carben-Metallkomplexe mit funktionellen Gruppen bzw. modifzierten funktionellen Gruppen exemplarisch in Schema 8 und 9 dargestellt.

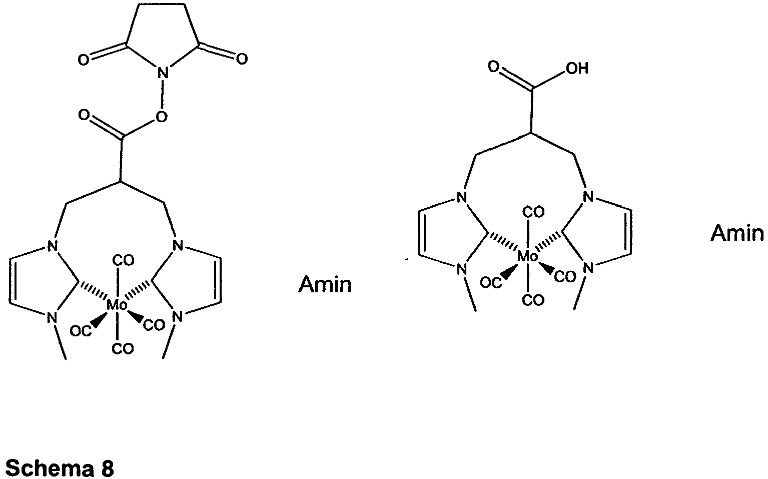

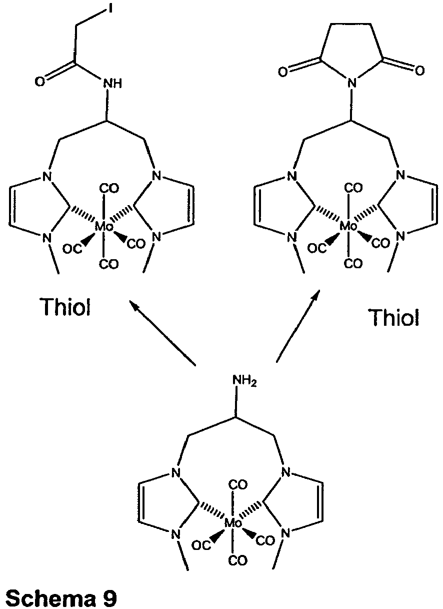

Die Bindung des jeweiligen Carben-Metallkomplexes kann beispielsweise auf einer Carbonsäure oder einem Amin als funktioneller Gruppe R³ basieren. Die Carbonsäure wird mit N-Succinamid umgesetzt. Anschließend erfolgt die Reaktion mit einer Thiol- oder Aminogruppe des jeweiligen Biomoleküls, was zu einer Thioether- oder Amidbindung führt. Eine Amingruppe als funktionelle Gruppe R³ kann zum lodacetamid umgesetzt werden, was nach Reaktion mit einer Biomolekül-Thiolgruppe zum Thioether führt. Ebenso kann die Amingruppe (R³) mit Bernsteinsäure zum Succinimid umgesetzt werden, welches wieder mit einer Thiol- oder Aminogruppe des Biomoleküls zu einer Thioether- oder Amidbindung führt. Vorzugsweise ist das Biomolekül über eine Thioether- oder Amidbindung kovalent an die funktionelle Gruppe R³ gebunden.

Für die Ankopplung an die Biomoleküle kann eine reverse Synthesestrategie eingesetzt werden, d.h. zuerst wird ein Imidazoliumsalz an das Biomolekül gebunden und anschließend wird das Metall in die Carbenvorstufe eingeführt. Auf diesem Weg kann ein breites Spektrum quasi "vormarkierter" Biomoleküle erzeugt werden, wobei über die Detektionsmethode später anhand der Auswahl des Metalls entschieden wird. Dies vereinfacht die Isolation, Aufreinigung sowie die Handhabung, da die Auswahl der Detektionsmethode bis zu einem Zeitpunkt unmittelbar vor der tatsächlichen Analyse aufgeschoben werden kann.

Die Prozedur über den normalen Weg, d.h. die Erzeugung des Carben-Metallkomplexes und anschließende Anbindung an das Biomolekül ist ebenfalls möglich. Beide Wege sind gangbar. Bei den Übergangscarbonyl-Vorstufen ist es aufgrund ihrer Anfälligkeit für Sauerstoff günstiger, zuerst die Carben-Metallkomplexe zu erzeugen und diese anschließend voll koordiniert an das Biomolekül zu binden.

Die Carben-Metallkomplexe finden Verwendung für die Analytik der Biomoleküle, wobei diese vorzugsweise als Ganzes oder in Teilen analysiert werden. Besonders bevorzugt werden die kovalent an ein Biomolekül gebundenen Carben-Metallkomplexe für den Transport von Metallen, vorzugsweise Technetium, Rhenium oder Kobalt, zu spezifischen Zielorganen (Radiomedizin) verwendet. Für den Transport sind die Bis- und Tris-, d.h. die einfachen Carben-Metallkomplexe sowie die Tetrakis-Systeme, d.h. die verbrückten Carben-Metallkomplexe, verwendbar.

### Literatur

[1] J. B. Fenn, M. Mann, C. K. Meng, S. F. Wong, C. M. Whitehouse, Science 246 (1989) 64
[2] L. A. Finney, T. V. O'Halloran, Science 300 (2003) 931.
[3] M. Wang, W. Y. Feng, Y. L. Zhao, Z. F. Chai, Mass Spectrom. Rev. 29 (2009) 326.
[4] J. Bettmer, N. Jakubowski, A. Prange, Anal. Bioanal. Chem. 386 (2006)7.
[5] A. Sanz-Medel, Anal. Bioanal. Chem. 391 (2008) 885.
[6] S. Bomke, M. Sperling, U. Karst, Anal. Bioanal. Chem. (2010) doi: 10.107/s00216-010-3611-1
[7] J. Szounar, Analyst 130 (2005) 442.
[8] N. Metzler-Nolte, Angew. Chem. 113 (2001) 1072.
[9] C. J. Koehler, M. Strozynski, F. Kozielski, A. Treumann, B. Thiede, J. Proteome Res. 8 (2009) 4333.
[10] L. Dayon, A. Hainard, V. Liecker, N. Turck, K. Kuhn, D. F. Hochstrasser, P. R. Burkhard, J.-C. Sanchez, Anal. Chem. 80 (2008) 2921.
[11] F. E. Hahn, M. Jahnke, Angew. Chem. Int. Ed. 47 (2008) 3122.
[12] O. Kühl, Chem. Soc. Rev. 36 (2007) 592.
[13] a) C. J. Adams, M. I. Bruce, P. Duckworth, P. A. Humphrey, O. Kühl, E. R. T. Tiekink, W. R. Cullen, P. Braunstein, S. Coco Cea, B. W. Skelton, A. H. White; J. Organomet. Chem. 467, 1994, 251; b) C. J. Adams, M. I. Bruce, O. Kühl, B. W. Skelton, A. H. White; J. Organomet. Chem. 445, 1993, C6; c) O. Kühl, M.Sc. Thesis, Tübingen 1992.
[14] O. Kühl, Coord. Chem. Rev. 249, 2005, 693.
[15] B. Seiwert, U. Karst, Anal. Bioanal. Chem. 390 (2008) 181.

Nachfolgend wird die Erfindung anhand von Beispielen weiter erläutert.

### Beispiele

### Beispiel 1: 3,3'-(2-(Methoxycarbonyl)propan-1,3-diyl)bis(1-methyl-imidazol-2-yliden)dibromid:

N-Methylimidazol (1,15 g, 14 mmol) wurde in Dioxan (15 mL) gelöst und mit Methyl-3-bromo-2-(bromomethyl) propionat (1,82 g, 7 mmol) versetzt. Die Reaktionslösung wurde für 6 Stunden unter Rühren auf 60°C erhitzt. Das Lösungsmittel wurde am Vakuum eingeengt und ein weißes Pulver erhalten, welches mit Hexan gewaschen wurde. Ausbeute: 2,948 g; 99 %.
¹H NMR (DMSO, ppm): ö : 3,63 (s, 3 H, OCH₃); 3,78 (quintett, ³J_{HH} = 6,3 Hz, 1 H, CH); 3,88 (s, 6 H, NCH₃); 4,54 (dd, ³J_{HH} = 6,5 Hz, ³J_{HH} = 1,4 Hz, 4 H, CH₂); 7,75 (dd, ³J_{HH} = 1,7 Hz, ⁴J_{HH} = 1,7 Hz, 2 H, H⁵); 7,79 (dd, ³J_{HH} = 1,7 Hz, ⁴J_{HH} = 1,7 Hz, 2 H, H⁴); 9,21 ppm (s, 2 H, H²)
¹³C NMR (DMSO, ppm): δ : 35,95 (NCH₃); 45,34 (CH₂); 47,13 (OCH₃); 52,72 (CH); 122,70 (C⁵); 123,79 (C⁴); 137,41 (C²); 169,95 (C=O).
Elementaranalyse: Berechnet für C₁₃H₂₀Br₂N₄O₂ (424,37 g·mol⁻¹): C 36,79 H 4,75 N 13,26; gefunden: C 36,70 5.29 H 13.30 N

### Beispiel 2: 3,3'-(2-(Methoxycarbonyl)propan-1,3-diyl)bis(1-(2,4,6-trimethylphenyl)-imidazol-2-yliden)dibromid

2,4,6-Trimethyl-N-imidazol (0,996 g,;5,35 mmol) und Methyl-3-bromo-2-(bromomethyl)propionat (0,695 g, 2,67 mmol) wurden in 15 ml Dioxan gelöst und unter Rühren für 8 Stunden auf 70°D erhitzt. Das Lösungsmittel wurde am Vakuum entfernt und ein dunkelgelbes, zähes Öl erhalten. Quantitative Ausbeute.
¹H NMR (DMSO, ppm): δ = 2,00 (s, 6 H, o-CH₃); 2,01 (s, 6 H, o-CH₃); 2,33 (s, 6 H, p-CH₃); 3,71 (s, 3 H, OCH₃); 7,15 (s, 4 H, Ar-H); 7,92 (dd, ³J_{HH} = 1,7 Hz, ⁴J_{HH} = 1,6 Hz, 1 H, H⁵); 7,96 (dd, ³J_{HH} = 1,8 Hz, ⁴J_{HH} = 1,7 Hz, 1 H, H⁵); 8,00 (dd, ³J_{HH} = 1,6 Hz, ⁴J_{HH} = 1,7 Hz, 1 H, H⁴); 8,04 (dd, ³J_{HH} = 1,8 Hz, ⁴J_{HH} = 1,7 Hz, 1 H, H⁴); 9,37 (dd, ⁴J_{HH} = 1,7 Hz, ⁴J_{HH} = 1,5 Hz, 1 H, H²); 9,55 (dd, ⁴J_{HH} = 1,5 Hz, ⁴J_{HH} = 1,3 Hz, 1 H, H²)
¹³C NMR (DMSO, ppm): δ : 16,82 (); 16,87 (); 20,58 (); 30,69 (); 44,81 (); 49,75 (); 52,33 (); 120,75 (); 123,19 (); 123,45 (); 124,09 ();-129,18 (); 129,24 (); 131,10 (); 131,26 (); 132,09 (); 133,46 (); 134,25 (); 134,34 (); 135,55 (); 138,17 (); 140,09 (); 140,29 (); 164,82 (C=O)

### Beispiel 3 : 2,2,2-Tris-N-Methylimidazol-essigsäure (Tris-COOH)

Es wurden 2.0 g Tribromessigsäure (6.73 mmol) mit 1,6 ml N-Methylimidazol (20,20 mmol) in 20 ml THF über Nacht im Kolben gerührt und anschließend das Lösungsmittel am Vakuum entfernt. Quantitative Ausbeute einer ionischen Flüßigkeit.
¹H NMR (DMSO, ppm): δ : 3.64 (s, 9 H, CH₃), 6.87 (s, 3 H, H⁵), 7.09 (s, 3 H, H⁴), 7.55 (s, 3 H, H²), 7.70 (s, 1 H, COOH)
¹³C NMR (DMSO, ppm): δ : 32.73 (CH₃), 120.45 (C⁵), 128.36 (C⁴), 137.86 (C²), quarternäres Kohlenstoffatome nicht detektiert.

### Beispiel 4: 2,2,2-Tris-N-Methylimidazol-essigsäureamid (Tris-CONH₂)

4.875 g (30 mmol) 2,2,2-Trichloroacetamid wurde in 40 ml THF gelöst und 7.2 ml (90 mmol) N-Methylimidazol unter Rühren zugegeben. Es wurde über Nacht gerührt und dann das Lösungsmittel am Vakuum entfernt. Ausbeute: 12.03 g; 98 %.
¹H NMR (DMSO, ppm): δ : 3.63 (s, 9 H, CH₃), 6.89 (s, 3 H, H⁵), 7.09 (s, 3 H, H⁴), 7.56 (s, 3 H, H²), 8.46 (s, 1 H, NH₂), 8.59 (s, 1 H, NH₂)
¹³C NMR (DMSO, ppm): δ : 32.77 (CH₃), 93.20 (C), 120.52 (C⁵), 128.41 (C⁴), 137.92 (C²), 163.09 (C=O)

### Beispiel 5: 2,2-Bis-N-Metylimidazol-essigsäureethylester (Bis-CO₂Et)

Es wurden 4,71 g Dichloressigsäure Ethylester (30 mmol) mit 4,8 ml N-Methylimidazol (60 mmol) in 20 ml THF über Nacht im Kolben gerührt und anschließend das Lösungsmittel am Vakuum entfernt. Quantitative Ausbeute einer ionischen Flüßigkeit.
¹H NMR (DMSO, ppm): ö : 1.25 (t, ³J_{HH} = 7.1 Hz, 3 H, CH₃), 3.64 (s, 6 H, NCH₃), 4.28 (qt, ³J_{HH} = 7.1 Hz, 2 H, CH₂), 6.88 (s, 1 H, CH), 6.89 (s, 2 H, H⁵), 7.11 (s, 2 H, H⁴), 7.59 (s, 2 H, H²)
¹³C NMR (DMSO, ppm): δ : 13.67 (CH₃), 32.79 (NCH₃), 63.47 (CH), 64.99 (CH₂), 120.50 (C⁵), 128.12 (C⁴), 137.80 (C²), 164.49 (C=O)

### Beispiel 6: [Ru(tris-COOH)(SOMe₂)Br₂]

287 mg Tris-COOH (0,545 mmol) wurden mit 379 mg (1,64 mmol) Ag₂O in 20 ml CH₂Cl₂ umgesetzt. Anschließend wurden 264 mg (0,545 mmol) [RuCl₂(DMSO)₄] zugesetzt und über Nacht gerührt. Die entstandene grüne Flüßigkeit wurde vom AgBr abfiltriert und am Vakuum eingeengt. Nach Zugabe von Hexan fällt eine grüne, luftstabile Verbindung aus, die in Wasser löslich ist und mit THF gereinigt wurde.
Ausbeute: 239 mg (0,374 mmol; 69%) [RuBr₂(DMSO)(Tris-COOH)].
MS (MALDI-tof): 642.359 [M+] (Isotope bei 638.360; 640.351; 642.359; 644.359; 646.350; 648.363), 598.402 [M+ - CO₂], 517.600 [M+ - CO₂ - ⁸¹Br]
IR [cm⁻¹]: 1954.36 (C=O).
Elementaranalyse für C₁₆H₂₂Br₂N₆O₃RuS (638.68): berechnet C 30.09 % H 3.47 % N 13.22 % ; gefunden C 29.52 % H 3.50 % N 12.67 %

### Beispiel 7: [Mo(Bis-CO₂Et)(CO)₄]

328,1 mg (1,02 mmol) Bis-CO₂Et wurden in 20 ml THF gelöst. Es wurden 696,5 mg (3 mmol) Ag₂O zugegeben. Die Suspension wurde über Nacht unter Lichtausschluss gerührt. Es wurden 296 mg (1,02 mmol) [Mo(CO)₄(NCMe₃)₂] zugegeben und über Nacht gerührt. Nach der Filtration wurde das Volumen im Vakuum eingeengt. Bei -20°C wurden farblose Kristalle erhalten.
Ausbeute: 191 mg (41 %)

### Beispiel 8: [Mo(Tris-CONH₂)(CO)₃]

409 mg (1 mmol) Tris-CONH₂ wurden in 20 ml CH₂Cl₂ gelöst und es wurden 367 mg (1,5 mmol) Ag₂O zugegeben. Die Suspension wurde über Nacht im Dunklen gerührt, anschließend wurden 290 mg (1 mmol) [Mo(CO)₄(NCCH₃)₂] zugegeben. Nach Rühren über Nacht wurde die Suspension filtriert und das Volumen des Filtrats im Vakuum eingeengt. Bei - 20°C wurden farblose Kristalle erhalten.
Ausbeute: 269 mg (47 %).

### Beispiel 9 ω,ω,ω-Tris-(N-methylimidazolyl)-buttersäure (0.25 THF)

994 mg (5.2 mmol) ω,ω,ω-Trichlorbuttersäure und 1.24 ml (15.6 mmol) N-Methylimidazol werden in 25 ml THF für 15 Stunden gerührt. Das Lösungsmittel wird im Vakuum entfernt. Das Produkt bleibt als farblose ionische Flüßigkeit zurück. Es enthält 0,25 Moleküle THF pro Formeleinheit.
Ausbeute: 2,4 g
¹H NMR (DMSO-d6, ppm): δ : 2.65 (m, 2H, ß-CH₂), 3.04 (m, 2H, □-CH₂), 3.64 (s, 9H, CH₃), 6.88 (s, 3H, H⁴), 7.10 (s, 3H, H⁵), 7.57 (s, 3H, H²).
¹³C NMR (DMSO-d6, ppm): δ : 31.29 (C-ß), 32.78 (CH₃), 49.54 (C-□), 99.43 (C), 120.50 (C⁴), 128.22 (C⁵), 137.84 (C²), 172.08 (C=O).

### Beispiel 10 ω,ωBis-(N-methylimidazolyl)-pentansäure

2 g (11,7 mmol) ω,ω-Dichloropentansüure und, 1.85 ml (23,4 mmol) N-methylimidazol werden in 30 ml THF für 15 Stunden gerührt. Das Lösungsmittel wird im Vakuum entfernt. Das Produkt bleibt als farblose ionische Flüßigkeit zurück.
Ausbeute: 3,9 g
¹H NMR (DMSO-d6, ppm): δ : 1.68 (p, 2H, ³J_{HH} = 7.4 Hz, ß-CH₂), 2.18 (m, 2H, γ-CH₂), 2.30 (t, 2H, ³J_{HH} = 7.4 Hz, □-CH₂), 3.64 (s, 6H, CH₃), 6.34 (t, 1H, ³J_{HH} = 5.9 Hz, CH), 6.89 (s, 2H, H⁴), 7.10 (d, 2H, ³J_{HH} = 1.0 Hz, H⁵), 7.59 (s, 2H, H²).
¹³C NMR (DMSO-d6, ppm): δ : 20.95 (C-ß), 32.26 (C-γ), 32.79 (CH₃), 42.14 (C-α), 74.52 (CH), 120.51 (C⁴), 128.09 (C⁵), 137.79 (C²), 174.02 (C=O).

### Beispiel 11 [Fe(ω,ω,ω-Tris-(N-methylimidazolyliden)-buttersäure)(PPh₃)Br₂]

623 mg (1.42 mmol) ω,ω,ω-Tris-(N-methylimidazolyl)-buttersäure und 598 mg (2.58 mmol) Ag₂O wurden für 16 Stunden in 30 ml Dichloromethan gerührt. Dann wurden 750 mg (1.42 mmol) [Fe(DMSO)₄Br₂] und 372 mg (1.42 mmol) PPh₃ zugesetzt. Nach 6 Stunden wurde die Suspension filtriert, das Filtrat am Vakuum eingeengt und Hexan zugesetzt. Es bildete sich ein rötlicher Niederschlag, der isoliert und getrocknet wurde. Ausbeute: 1.04 g (1.28 mmol, 90 %).
MS (MALDI-tof) 808 [M+]
³¹P NMR (DMSO-d6, ppm): δ : 7.88

## Patentansprüche

1. Imidazoliumsalze der allgemeinen Formel I, 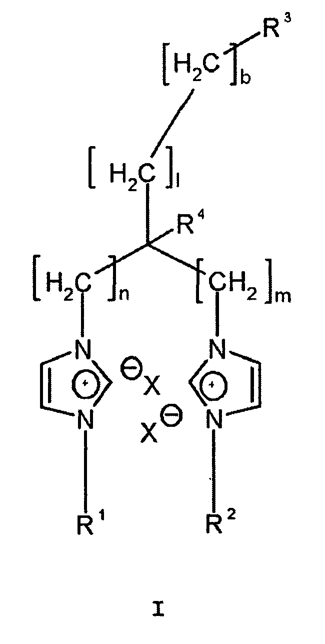 wobei b, I, m, n und gegebenenfalls q gleich oder verschieden sind und jeweils unabhängig voneinander Null oder 1 sind; und
X⁻ ein Anion ist;
R¹ und R² gleich oder verschieden sind und offenkettig, verzweigt oder unverzweigt oder als Bestandteil eines Ringes oder Ringsystems vorliegen und ausgewählt sind aus der Gruppe von Wasserstoff, C₁-C₁₂-n-Alkylrest, C₃-C₁₂-verzweigter Alkylrest, C₃-C₁₂-Cycloalkylrest, C₂-C₁₂-Alkenylrest, C₇-C₂₀-Arylalkylrest, C₅-C₁₄-Arylrest,
welcher seinerseits gleich oder unterschiedlich substituiert ist durch einen oder mehrere verzweigte oder unverzweigte C₁-C₃-Alkylreste, wobei die cyclischen und aromatischen Systeme eingliedrige Ringe oder mehrgliedrige annelierte oder isolierte Ringe sind oder mit dem Imidazolring der Struktur gemäß Formel I ein Ringsystem bilden;
R³ ausgewählt ist aus der Gruppe von -NH₂, -COOH, -CONH₂ und -COOR"-Rest, wobei R" ein C₁-C₆-n-Alkylrest ist;
R⁴ Wasserstoff oder R⁵ ist, wobei R⁵ ein Imidazolring der Formel II ist, 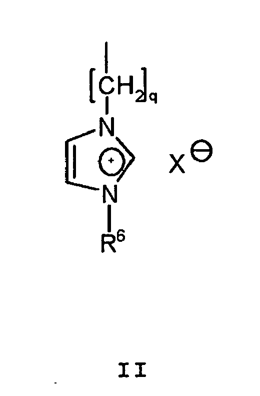 wobei q Null oder 1 ist;
R⁶ gleich oder verschieden zu R¹ und R² und aus der gleichen Gruppe wie R¹, R² ausgewählt ist.

2. Imidazoliumsalze nach Anspruch 1, **dadurch gekennzeichnet, dass** die Reste R¹, R² und gegebenenfalls R⁶ gleich oder verschieden sind und ausgewählt sind aus der Gruppe der C₁-C₆-n-Alkylreste.

3. Imidazoliumsalze nach einem der Ansprüche 1 bis 2, **dadurch gekennzeichnet, dass** n, m und gegebenenfalls q Null oder 1 sowie b und I Null sind.

4. Imidazoliumsalze nach einem der Ansprüche 1 bis 3, **dadurch gekennzeichnet, dass** R" ein C₁-C₆-n-Alkylrest ist.

5. Imidazoliumsalze gemäß der allgemeinen Formel III, 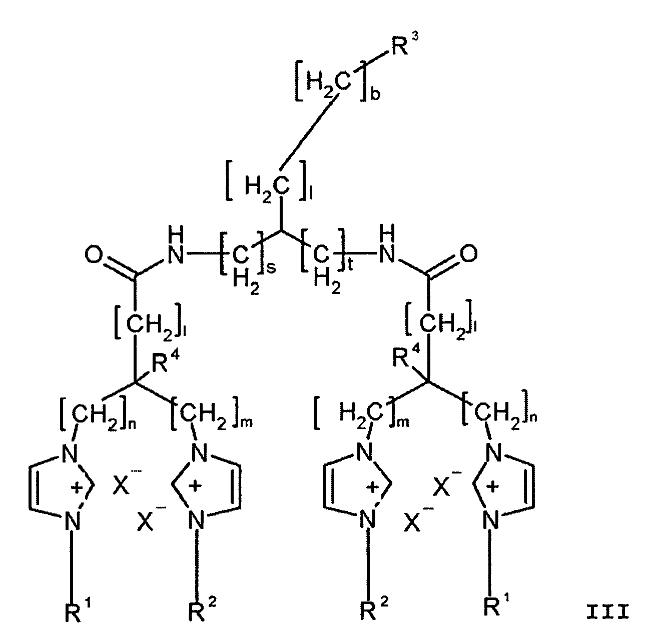 wobei R¹ bis R⁴ sowie b, l, m, n die in einem der Ansprüche 1-4 genannte Bedeutung haben, sowie s und t gleich oder verschieden sind und jeweils unabhängig voneinander Null oder eine ganze Zahl von 1 bis 3 sind.

6. Carben-Metallkomplexe der allgemeinen Formel IV 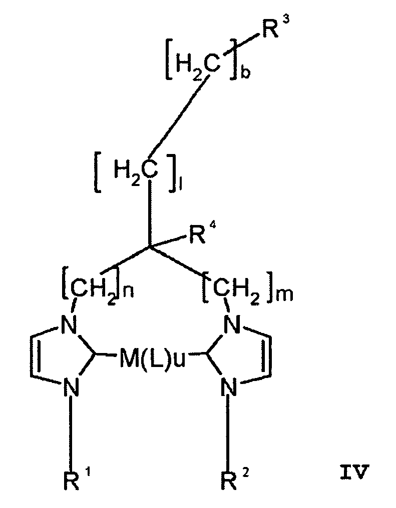 wobei R¹ bis R⁴ und gegebenenfalls R⁶ und b, l, m, n und gegebenenfalls q die in einem der Ansprüche 1-5 genannte Bedeutung haben und M ein Metall der 6.-12. Gruppe des Periodensystems, L ein Ligand und u eine ganze Zahl von 1 bis 4 ist, wobei bei u = 2, 3 oder 4 die Liganden L gleich oder verschieden sind und mehrere Liganden L gegebenenfalls miteinander einen zwei- oder mehrzähnigen Chelatliganden bilden und wobei bei R⁴= Imidazolring gemäß Formel II der Imidazolring ebenfalls an das Metall M koordiniert.

7. Carben-Metallkomplexe nach einem der Ansprüche 5 oder 6, **dadurch gekennzeichnet, dass** das Metall M aus der Gruppe der Metalle Kupfer, Eisen, Ruthenium, Nickel und Palladium oder aus der Gruppe der Metalle Technetium, Rhenium und Kobalt ausgewählt ist.

8. Carben-Metallkomplexe nach einem der Ansprüche 5 bis 9, **dadurch gekennzeichnet, dass** L ausgewählt ist aus der Gruppe von
CO;
Nitril;
Isonitril;
Nitrosyl;
Halogenidion;
Wasserstoffatom;
C₁-C₁₂-Alkylanion, Allylanion, Methallylanion, Benzylanion, C₆-C₁₄-Arylanion, C₁-C₁₂-Alkoxyanion, C₆-C₁₄-Aryloxyanion, C₁-C₁₂-Heteroalkylanion, C₆-C₁₄-Heteroarylanion, C₁-C₁₂-Heteroalkoxyanion, C₆-C₁₄-Heteroaryloxyanion oder C₆-C₁₄-Heteroaryloxyanion, welches unsubstituiert ist oder gleich oder unterschiedlich substituiert ist durch ein oder mehrere C₁-C₁₂-Alkylreste oder C₁-C₁₂-Heteroalkylreste, wobei die Heteroatome aus der Gruppe von B, Al, Ga, In, N, P, As, Sb, Bi, Si, Ge, Sn, Pb, O, S, Se und Te ausgewählt sind und wobei die Anionen unsubstituiert oder ganz oder teilweise mit einem oder mehreren, gleichen oder unterschiedlichen Heteroatomen aus der selben Gruppe in Form funktioneller Gruppe(n) substituiert sind, wobei die cyclischen und aromatischen Systeme eingliedrige Ringe oder mehrgliedrige annelierte oder isolierte Ringe sind; primäres, sekundäres, tertiäres Amin;
primäres, sekundäres, tertiäres Phosphan;
primäres, sekundäres, tertiäres Phosphit oder Carben.

9. Carben-Metallkomplexe nach einem der Ansprüche 5 bis 8, **dadurch gekennzeichnet, dass** L ausgewählt ist aus der Gruppe der Halogenidionen, vorzugsweise Bromid oder Chlorid.

10. Carben-Metallkomplexe der allgemeinen Formel V 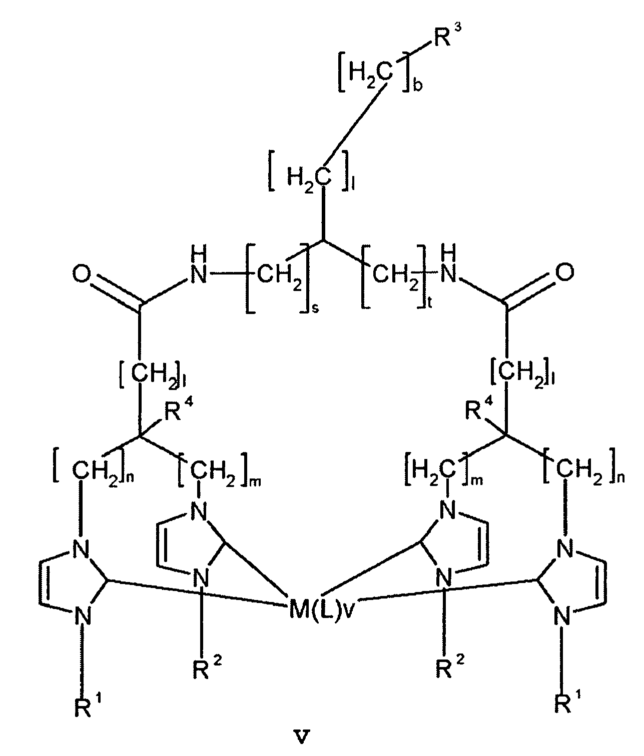 wobei R¹ bis R³ und b, l, m und n sowie M und L die in einem der Ansprüche 5-9 genannte Bedeutung haben, R⁴ Wasserstoff ist und v Null, 1 oder 2 ist.

11. Carben-Metallkomplexe gemäß den Ansprüchen 5 bis 10 zur Verwendung als bioanalytische Marker für Biomoleküle, wobei die Metallkomplexe über die funktionelle Gruppe des Restes R³ kovalent an eine entsprechende funktionelle Gruppe des Biomoleküls, vorzugsweise eine Hydroxyl-, Thiol- oder Amino-Gruppe, besonders bevorzugt über eine Thiol- oder Amino-Gruppe, gebunden sind.

12. Carben-Metallkomplexe gemäß Anspruch 11 zur Verwendung als bioanalytische Marker für Biomoleküle, wobei das Biomolekül ausgewählt ist aus der Gruppe von Proteinen, Peptiden, Kohlenhydraten, Nukleinbasen, DNA und RNA.

13. Carben-Metallkomplexe gemäß einem der Ansprüche 11 oder 12 zur Verwendung als bioanalytische Marker für Biomoleküle, wobei die Bindung an das Biomolekül eine Thioether- oder Amidbindung ist.

14. Carben-Metallkomplexe gemäß einem der Ansprüche 11 bis 13 zur Verwendung als bioanalytische Marker für Biomoleküle, für die Analytik der Biomoleküle, wobei die Biomoleküle als Ganzes oder in Teilen analysiert werden.

15. Kovalent an ein Biomolekül gebundenen Carben-Metallkomplexe gemäß einem der Ansprüche 11 bis 14 zur Verwendung als bioanalytische Marker für Biomoleküle für den Transport von Metallen, vorzugsweise Technetium, Rhenium oder Kobalt, zu spezifischen Zielorganen.

## Claims

1. Imidazolium salts of the general formula I, 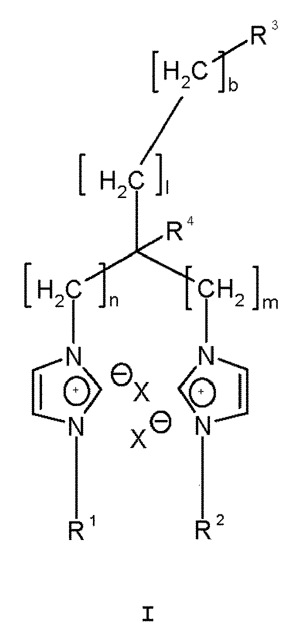 wherein b, l, m, n and q, where applicable, are the same or different and each independent of each other zero or 1; and
X⁻ is an anion;
R¹ and R² are the same or different and open chained, branched or unbranched or are part of a ring or a ring system and are chosen from the group of hydrogen, C₁-C₁₂-n-alkyl, C₃-C₁₂-branched alkyl, C₃-C₁₂-cycloalkyl, C₂-C₁₂-alkenyl, C₇-C₂₀-arylalkyl, C₅-C₁₄-aryl,
which itself is either the same or differently substituted by one or more branched or unbranched C₁-C₃-alkyl residues, wherein the cyclic and aromatic systems are single rings or several condensed, annelated or isolated rings or form a ring system together with the imidazolium ring of the structure according to formula I;
R³ is chosen from the group of -NH₂, -COOH, -CONH₂ and -COOR" residue, wherein R" is a C₁-C₆-n-alkyl residue;
R⁴ is hydrogen or R⁵, wherein R⁵ is an imidazolium ring of formula II, 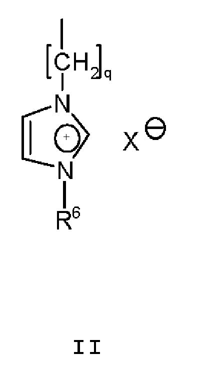 wherein q is zero or 1;
R⁶ is the same or different to R¹ and R² and chosen from the same group as R¹, R².

2. Imidazolium salts according to claim 1, wherein the residues R¹, R² and R⁶, where applicable, are the same or different and chosen from the group of C₁-C₆-n-alkyl residues.

3. Imidazolium salts according to one of the claims 1 or 2, wherein n, m and q, where applicable, are zero or 1 as well as b and I are zero.

4. Imidazolium salts according to one of the claims 1 to 3, wherein R" is a C₁-C₆-n-alkyl residue.

5. Imidazolium salts according to the general formula III, 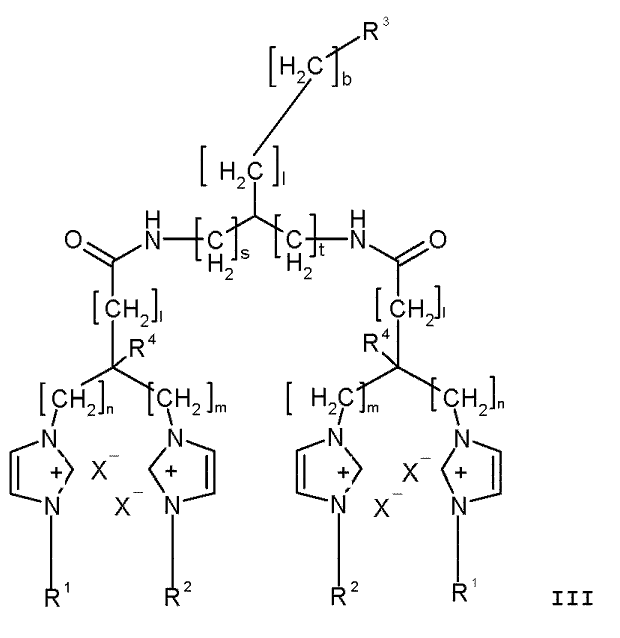 wherein R¹ to R⁴ as well as b, l, m, n have the meaning given in one of the claims 1 to 4 and s and t are the same or different and are independently of each other zero or an integer number from 1 to 3.

6. Carbene metal complexes of the general formula IV, 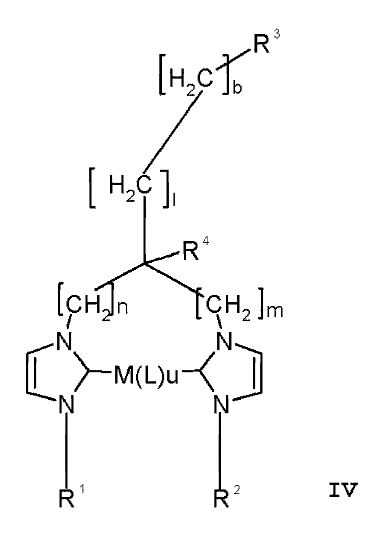 wherein R¹ to R⁴ and R⁶, where applicable, and b, l, m, n and q, where applicable, have the meaning given in one of the claims 1-5 and M is a metal of the 6. - 12. group of the Periodic Table of the Elements, L is a ligand and u is an integer number from 1 to 4, whereby if u = 2, 3 or 4, the ligands L are either the same or different and several ligands L, where applicable, form a bi- or polydentate chelate ligand and wherein if R⁴ = imidazole ring according to formula II, the imidazole ring also coordinates to the metal M.

7. Carbene metal complexes according to claim 6, wherein the metal is chosen from the group of metals copper, iron, ruthenium, nickel and palladium or from the group of metals technetium, rhenium and cobalt.

8. Carbene metal complexes according to one of the claims 6 to 7, wherein the ligand L is chosen from the group
CO;
nitrile;
isonitrile;
nitrosyl;
halogenide ion;
hydrogen atom;
C₁-C₁₂-alkyl anion, allyl anion, methylallyl anion, benzyl anion, C₆-C₁₄-aryl anion, C₁-C₁₂-alkoxy anion, C₆-C₁₄-aryloxy anion, C₁-C₁₂-heteroalkyl anion, C₆-C₁₄-heteroaryl anion, C₁-C₁₂-heteroalkoxy anion, C₆-C₁₄-heteroaryloxy anion or C₆-C₁₄-heteroaryloxy anion, which is unsubstituted or the same or differently substituted by one or more C₁-C₁₂-alkyl residues or C₁-C₁₂-heteroalkyl residues, whereby the heteroatoms are chosen from the group B, Al, Ga, In, N, P, As, Sb, Bi, Si, Ge, Sn, Pb, O, S, Se and Te and wherein the anions are unsubstituted or wholly or partially substituted with one or more than one, same or different heteroatoms from the same group, in the form of functional groups, wherein the cyclic and aromatic systems are single rings or several annelated or isolated rings;
primary, secondary, tertiary amine;
primary, secondary, tertiary phosphane;
primary, secondary, tertiary phosphite or carbene.

9. Carbene metal complexes according to one of the claims 6 to 8, wherein the ligand L is chosen from the group of halogenide ions, preferably bromide or chloride.

10. Carbene metal complexes of the general formula V, 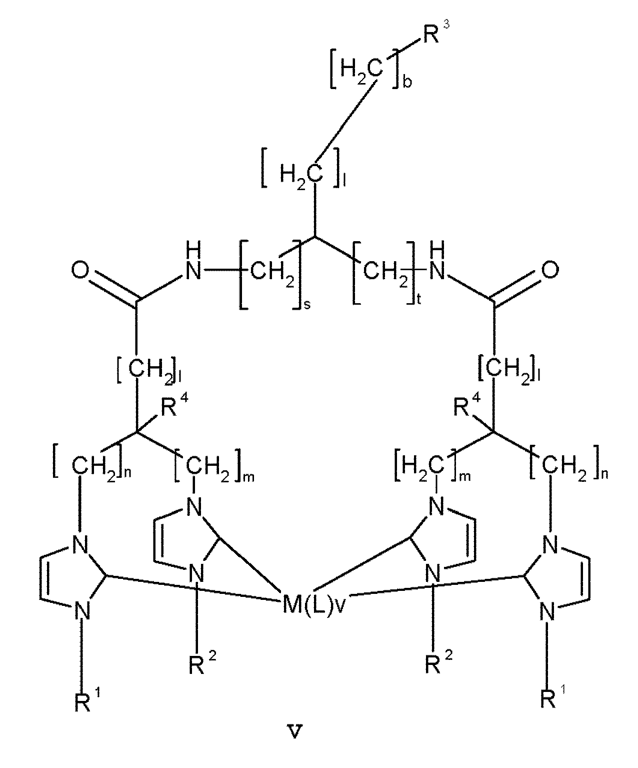 wherein R¹ to R³ and b, l, m and n as well as M and L have the meaning given in one of the claims 5-9, R⁴ is hydrogen and v is zero, 1 or 2.

11. Carbene metal complexes according to the claims 6 to 10 for use as bioanalytical tags for biomolecules, wherein the metal complexes are covalently bonded with the functional residue R³ to the respective functional group of the biomolecule, preferably a hydroxyl, thiol or amino group, particularly preferred a thiol or amino group.

12. Carbene metal complexes according to claim 11 for use as bioanalytical tags for biomolecules, wherein the biomolecule is chosen from the group of proteins, peptides, carbohydrates, nuclein bases, DNA and RNA.

13. Carbene metal complexes according to one of the claims 11 or 12 for use as bioanalytical tags for biomolecules, wherein the bond to the biomolecule is a thioether or an amide bond.

14. Carbene metal complexes according to the claims 11 to 13 for use as bioanalytical tags for biomolecules, for the analysis of the biomolecules, wherein the biomolecules are analysed as a whole or in parts.

15. Carbene metal complexes covalently bonded to a biomolecule according to one of the claims 11 to 14 for use as bioanalytical tags for biomolecules for the transport of metals, preferably technetium, rhenium or cobalt, to specific target organs.

## Revendications

1. Sel d'imidazolium de la formule générale I, 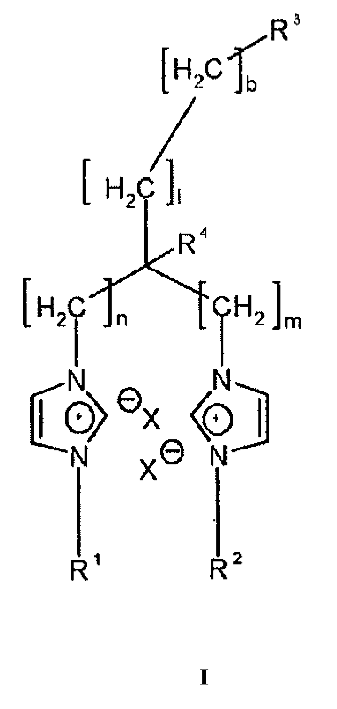 dans laquelle b, l, m, n et le cas échéant q sont identiques ou différents et chaque fois indépendamment les uns des autres sont égaux à zéro ou 1 ; et X' est un anion ;
R¹ et R² sont identiques ou différents et se présentent en chaîne ouverte, ramifiés ou non ramifiés ou en tant qu'élément d'un composé cyclique ou d'une combinaison cyclique et sont choisis dans le groupe comprenant l'hydrogène, un radical n-alkyle en C₁ à C₁₂, un radical alkyle ramifié en C₃ à C₁₂, un radical cycloalkyle en C₃ à C₁₂, un radical alkényle en C₂ à C₁₂, un radical arylalkyle en C₇ à C₂₀, un radical aryle en C₅ à C₁₄,
lequel, pour sa part est substitué à l'identique ou différemment par un ou plusieurs radicaux alkyle en C₁ à C₃ ramifiés ou non ramifiés, les combinaisons cycliques et aromatiques étant des composés cycliques à un chaînon ou des composés cycliques condensés ou isolés à un ou à plusieurs chainons ou formant avec le cycle imidazole de la structure selon la formule I une combinaison cyclique ;
R³ est choisi dans le groupe comprenant un radical NH2, COOH, CONH₂ et COOR",
R" étant un radical n-alkyle en C₁ à C₆ ;
R⁴ étant de l'hydrogène ou R⁵, R⁵ étant un cycle imidazole de la formule II, 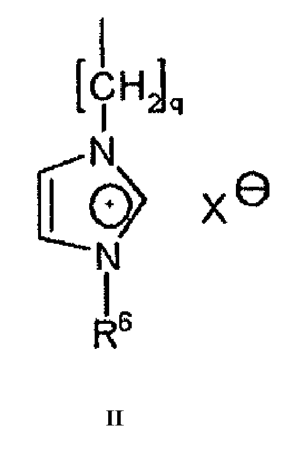 q étant égal à zéro ou à 1 ;
R⁶ étant égal ou différent de R¹ et de R² et étant choisi dans le même groupe que R¹, R².

2. Sels d'imidazolium selon la revendication 1, **caractérisés en ce que** les radicaux R¹, R² et le cas échéant R⁶ sont identiques ou différents et sont choisis dans le groupe des radicaux n-alkyle en C₁ à C₆.

3. Sels d'imidazolium selon l'une quelconque des revendications 1 à 2, **caractérisés en ce que** n, m et le cas échéant q sont égaux à zéro ou à 1 et b et l sont égaux à zéro.

4. Sels d'imidazolium selon l'une quelconque des revendications 1 à 3, **caractérisés en ce que** R" est un radical n-alkyle en C₁ à C₆.

5. Sels d'imidazolium selon la formule générale III, 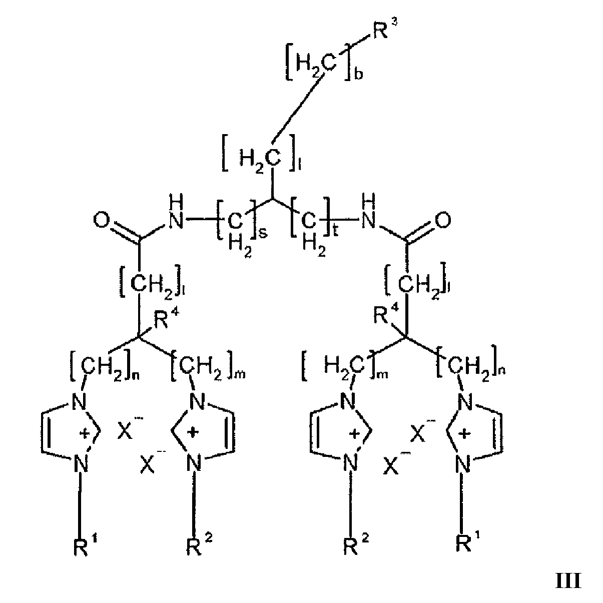 R¹ et R⁴, ainsi que b, l, m, n ayant la signification citée dans l'une des revendications 1 à 4, et s et t étant identiques ou différents et chaque fois indépendamment l'un de l'autre étant égaux à zéro ou à un nombre entier de 1 à 3.

6. Complexes carbène-métal de la formule générale IV 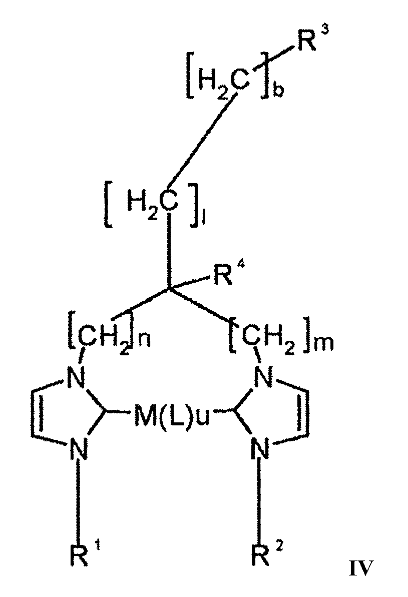 R¹ à R⁴ et le cas échéant R⁶ et b, l, m, n et le cas échéant q ayant la signification citée dans l'une des revendications 1 à 5 et M étant un métal du 6^{ème} au 12^{ème} groupe, L étant un ligand et u étant un nombre entier de 1 à 4, lorsque u = 2, 3 ou 4, les ligands L étant identiques ou différents et plusieurs ligands L formant ensemble le cas échéant un ligand chélate bidenté ou polydenté et lorsque R⁴= cycle imidazole selon la formule II, le cycle imidazole se coordonnant également au métal M.

7. Complexes carbène-métal selon la revendication 6, **caractérisé en ce que** le métal M est choisi dans le groupe des métaux cuivre, fer, ruthénium, nickel et palladium ou dans le groupe des métaux technétium, rhénium et cobalt.

8. Complexes carbène-métal selon l'une quelconque des revendications 6 à 7, **caractérisé en ce que** L est choisi dans le groupe comprenant le CO ;
un nitrile ;
un isonitrile ;
un nitrosyle ;
un ion halogénure ;
un atome d'hydrogène ;
un anion alkyle en C₁ à C₁₂, un anion allyle, un anion methallyle, un anion benzyle, un anion aryle en C₆ à C₁₄, un anion alcoxy en C₁ à C₁₂, un anion aryloxy en C₆ à C₁₄, un anion hétéroalkyle en C₁ à C₁₂, un anion hétéroaryle en C₆ à C₁₄, un anion hétéroalkoxy en C₁ à C₁₂, un anion hétéroaryloxy en C₆ à C₁₄ ou un anion hétéroaryloxy en C₆ à C₁₄, lequel est non substitué ou substitué de manière identique ou différente par un ou plusieurs radicaux alkyle en C₁ à C₁₂ ou radicaux hétéroalkyle en C₁ à C₁₂, les hétéroatomes étant choisis dans le groupe comprenant B, Al, Ga, In, N, P, As, Sb, Bi, Si, Ge, Sn, Pb, O, S, Se et Te et les anions étant non substitués ou étant substitués entièrement ou en partie avec un ou plusieurs hétéroatomes identiques ou différents du même groupe sous la forme d'un (de) groupe(s) fonctionnel(s), les combinaisons cycliques et aromatiques étant des composés cycliques à un chaînon ou des composés cycliques condensés ou isolés à plusieurs chainons ;
une amine primaire, secondaire ou tertiaire ;
un phosphane primaire, secondaire, tertiaire ;
un phosphite ou un carbène primaire, secondaire, tertiaire.

9. Complexes carbène-métal selon l'une quelconque des revendications 6 à 8, **caractérisé en ce que** L est choisi dans le groupe des ions halogénure, de préférence le bromure ou le chlorure.

10. Complexes carbène-métal de la formule générale V 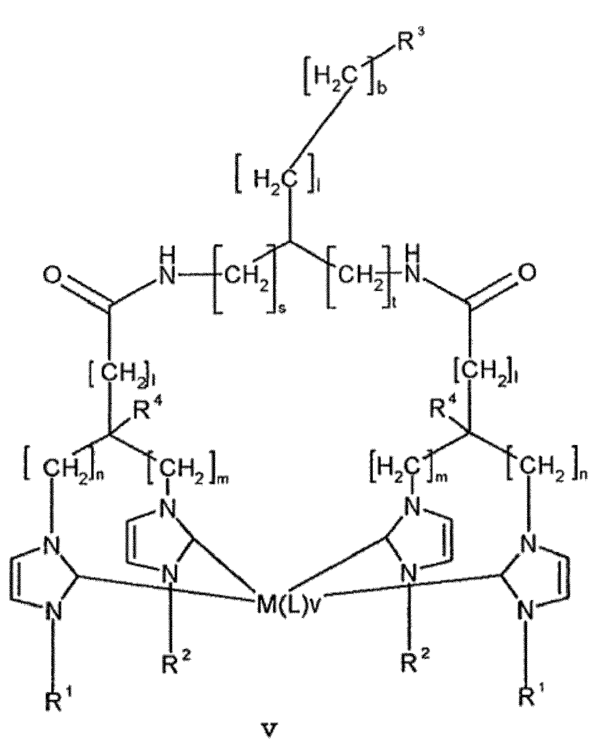 R¹ à R³ et b, l, m et n, ainsi que M et L ayant la signification citée dans l'une des revendications 6 à 9, R⁴ étant de l'hydrogène et v étant égal à zéro, 1 ou 2.

11. Complexes carbène-métal selon les revendications 6 à 10, destinés à être utilisés en tant que marqueurs bio-analytiques pour des biomolécules, les complexes carbène-métal étant liés de manière covalente par l'intermédiaire du groupe fonctionnel du radical R³ à un groupe fonctionnel correspondant de la biomolécule, de préférence un groupe hydroxyle, un groupe thiol ou un groupe amino, de manière particulièrement préférée par l'intermédiaire d'un groupe thiol ou d'un groupe amino.

12. Complexes carbène-métal selon la revendication 11, destinés à être utilisés en tant que marqueurs bio-analytiques pour des biomolécules, la biomolécule étant choisie dans le groupe comprenant les protéines, les peptides, les hydrates de carbone, les bases nucléiques, l'ADN et l'ARN.

13. Complexes carbène-métal selon l'une quelconque des revendications 11 ou 12, destinés à être utilisés en tant que marqueurs bio-analytiques pour des biomolécules, la liaison à la biomolécule étant une liaison thioéther ou une liaison amide.

14. Complexes carbène-métal selon l'une quelconque des revendications 11 à 13, destinés à être utilisés en tant que marqueurs bio-analytiques pour des biomolécules, pour l'analyse des biomolécules, les biomolécules étant analysées en tant qu'entité ou par éléments.

15. Complexes carbène-métal liés à une biomolécule selon l'une quelconque des revendications 11 à 14, destinés à être utilisés en tant que marqueurs bio-analytiques pour des biomolécules pour le transport de métaux, de préférence de technétium, de rhénium ou de cobalt, vers des organes cibles spécifiques.
